(19) Europäisches Patentamt
European Patent Office
Office européen des brevets

(11) Veröffentlichungsnummer: **0 204 659 B1**

# (12) EUROPÄISCHE PATENTSCHRIFT

(45) Veröffentlichungstag der Patentschrift: **21.11.91**

(51) Int. Cl.$^{5}$: **C08G 59/02**, C08L 63/00, C09J 163/00, H01L 21/56

(21) Anmeldenummer: **86810235.1**

(22) Anmeldetag: **02.06.86**

## (54) Polyepoxide und deren Verwendung.

(30) Priorität: **06.06.85 CH 2399/85**

(43) Veröffentlichungstag der Anmeldung: **10.12.86 Patentblatt 86/50**

(45) Bekanntmachung des Hinweises auf die Patenterteilung: **21.11.91 Patentblatt 91/47**

(84) Benannte Vertragsstaaten: **CH DE FR GB IT LI NL**

(56) Entgegenhaltungen:
**EP-A- 0 013 258**
**FR-A- 2 355 047**
**GB-A- 828 364**

(73) Patentinhaber: **CIBA-GEIGY AG**
**Klybeckstrasse 141**
**CH-4002 Basel(CH)**

(72) Erfinder: **Monnier, Charles E., Dr.**
**Ch. du Verger 16**
**CH-1752 Villars-sur-Glâne(CH)**
Erfinder: **Zahir, Sheik Abdul-Cader, Dr.**
**Elsternstrasse 12**
**CH-4104 Oberwil(CH)**

**EP 0 204 659 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft neue Polyepoxide, solche Polyepoxide enthaltende härtbare Gemische und deren Verwendung, z.B. zur Herstellung von gehärteten Produkten, besonders zum Umhüllen elektronischer Bauteile, oder als Klebstoffe.

In der britischen Patentschrift Nr. 828.364 sind unter anderem höhermolekulare Polyepoxide beschrieben, die durch Umsetzung von o,o'-diallylsubstituiertem Bisphenol A und Bisphenol-A-Diglycidylether und anschliessende Epoxidierung der erhaltenen Addukte mit Persäuren hergestellt werden.

Gegenstand der Erfindung sind neue Polyepoxide, die dadurch erhältlich sind, dass man Verbindungen der Formel I

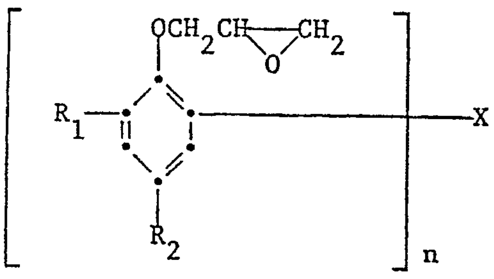

(I),

worin n die Zahl 1 oder 2 ist, X bei n = 1 eine Gruppe $R_3$ und bei n = 2 eine Gruppe der Formel

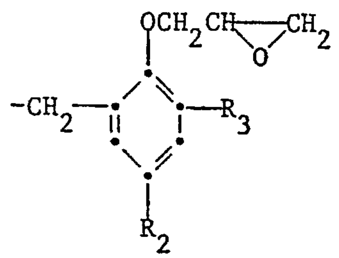

darstellt,

$R_1$ und $R_3$ unabhängig voneinander $C_{1-4}$-Alkyl, $C_{1-4}$-Alkoxy, Allyl, ein Halogenatom oder $C_{6-10}$-Aryl und $R_2$ Allyl oder 1-Propenyl bedeuten, mit Verbindungen mit phenolischen OH-Gruppen umsetzt und die erhaltenen Addukte anschliessend in Gegenwart einer Persäure epoxidiert.

Alkyl- und Alkoxysubstituenten $R_1$ und $R_3$ können geradkettig oder verzweigt sein. Beispiele solcher Gruppen sind: Methyl, Ethyl, n-Propyl, Isopropyl, n-Butyl, sek.-Butyl, tert.-Butyl, Methoxy, Ethoxy, n-Propoxy, Isopropoxy, n-Butoxy und sek.-Butoxy. Als Arylgruppen $R_1$ und/oder $R_3$ kommen z.B. 1-Naphthyl, 2-Naphthyl und besonders Phenyl in Betracht. Stellen $R_1$ und/oder $R_3$ Halogenatome dar, so handelt es sich z.B. um Brom- oder Fluoratome und besonders um Chloratome.

Bevorzugt verwendet man Verbindungen der Formel I, worin $R_1$ und $R_3$ unabhängig voneinander $C_{1-4}$-Alkyl, $C_{1-2}$-Alkoxy, ein Halogenatom, besonders ein Chloratom, oder Phenyl und $R_2$ Allyl darstellen. Gemäss einer weiteren Bevorzugung verwendet man Verbindungen der Formel I, worin $R_1$ und $R_3$ dieselbe Bedeutung haben und $R_2$ Allyl ist. n ist vorzugsweise 1. Besonders bevorzugt setzt man Verbindungen der Formel I ein, worin n = 1 ist, $R_1$ und $R_3$ je Methyl, tert.-Butyl, Methoxy, Chlor oder Phenyl und $R_2$ Allyl bedeuten. Ganz besonders bevorzugt sind Verbindungen der Formel I, worin n = 1 ist, $R_1$ und $R_3$ je Methyl und $R_2$ Allyl darstellen.

Als Verbindungen mit phenolischen OH-Gruppen zur Herstellung der Addukte eignen sich z.B. Mono- und Polyphenole, die ein- oder mehrkernig sein können, sowie Umsetzungsprodukte aus Polyepoxiden und einem Ueberschuss an Polyphenolen. Als Beispiele von Mono- und Polyphenolen seien genannt: Phenol, Toluol, Xylole, 2-Allylphenol, 2,6-Diallylphenol, Triallylphenol, Rescorcin, Hydrochinon, Bis(4-hydroxyphenyl)methan (Bisphenol F), 2,2-Bis(4-hydroxyphenyl)propan (Bisphenol A), 2,2-Bis(4-hydroxy-3,5-dibromphenyl)propan (Tetrabrombisphenol A), 2,2-Bis(4-hydroxy-3-allylphenyl)propan, 2,2-Bis(4-hydroxy-3,5-diallylphenyl)propan, Bis(2-hydroxy-3-methyl-5-allylphenyl)methan, 2,2-Bis(4-hydroxy-3-propenylphenyl)propan, 1,1,2,2-Tetrakis(4-hydroxyphenyl)ethan, 4,4'-Dihydroxybiphenyl, Bis(4-hydroxyphenyl)sulfon und Novolake aus Formaldehyd oder Acetaldehyd und Phenol, Chlorophenol, Allylphenol oder Alkylphenolen mit zu 9 C-Atomen im Alkyl. Besonders bevorzugt als Mono- und Polyphenole sind Bisphenol A, Bisphenol F, Tetrabrombisphenol A, Triallylphenol, 2,2-Bis(4-hydroxy-3-allylphenyl)propan, 2,2-Bis(4-hydroxy-3,5-diallylphenyl)propan, Phenolformaldehyd- und Kresolformaldehyd-Novolake.

Für die Umsetzungsprodukte aus Polyepoxiden und einem Ueberschuss an Polyphenole verwendet man bevorzugt ein- oder zweikernige Verbindungen der oben genannten Art und Epoxidharze mit durchschnittlich mehr als einer an ein Heteroatom, z.B. an ein S- und vorzugsweise an ein O- oder N-Atom, gebundenen Gruppe der Formel II

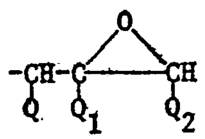

(II),

worin Q und $Q_2$ je ein Wasserstoffatom und $Q_1$ ein Wasserstoffatom oder eine Methylgruppe oder Q und $Q_2$ zusammen $-CH_2CH_2-$ oder $-CH_2CH_2CH_2-$und $Q_1$ ein Wasserstoffatom darstellen.

Als Beispiele solcher Polyepoxide seien Polyglycidyl- und Poly($\beta$-methylglycidyl)ester genannt, die sich von aliphatischen, cycloaliphatischen oder aromatischen Polycarbonsäuren ableiten. Bespiele geeigneter Polycarbonsäuren sind Bernsteinsäure, Glutarsäure, Adipinsäure, Pimelinsäure, Korksäure, Azelainsäure, Sebacinsäure, dimerisierte oder trimerisierte Linolsäure, Tetrahydrophthalsäure, 4-Methyltetrahydrophthalsäure, Hexahydrophthalsäure, 4-Methylhexahydrophthalsäure, Phthalsäure, Iso- und Terephthalsäure.

Weitere Beispiele sind Polyglycidyl- und Poly($\beta$-methyglycidyl)-ether, die durch Umsetzung einer mindestens zwei alkoholische und/oder phenolische Hydroxylgruppen pro Molekül enthaltenden Verbindung mit Epichlorhydrin oder mit Allylchlorid und anschliessende Epoxidierung mit Persäuren erhalten werden. Geeignete Polyole sind z.B. Ethylenglykol, Diethylenglykol, Poly(oxyethylen)glykole, Propan-1,2-diol, Poly-(oxypropylen)glykole, Propan-1,3-diol, Butan-1,4-diol, Poly(oxytetramethylen)glykole, Pentan-1,5-diol, Hexan-2,4,6-triol, Glycerin, 1,1,1-Trimethylolpropan, Pentaerythrit und Sorbit; 1,3- und 1,4-Cyclohexandiol, Bis(4-hydroxycyclohexyl)methan, 2,2-Bis(4-hydroxycyclohexyl)propan; ferner aromatische Polyole der oben aufgezählten Art.

Als Poly(N-glycidyl)-Verbindungen kommen durch Dehydrochlorierung von Umsetzungsprodukten aus Epichlorhydrin und Aminen mit mindestens zwei Aminwasserstoffatomen erhaltene Produkte in Betracht. Geeignete Amine sind z.B. Anilin, n-Butylamin, Bis-(4-aminophenyl)methan, 1,3- und 1,4-Xylylendiamin, 1,3- und 1,4-Bis(aminomethyl)cyclohexan und Bis(4-methylaminophenyl)methan. Triglycidylisocyanurat, N,N'-Diglycidylderivate von cyclischen Alkylenharnstoffen, wie Ethylenharnstoff und 1,3-Propylenharnstoff, oder Hydantoinen, wie 5,5-Dimethylhydantoin, sind weitere geeignete derartige Verbindungen.

Poly(S-glycidyl)-Verbindungen sind z.B. die Di-S-Glycidylderivate von Dithiolen, wie Ethan-1,2-dithiol und Bis-(4-mercaptomethylphenyl)ether.

Beispiele für Epoxidharze mit einer oder mehreren Gruppen der Formel II, in welcher Q und $Q_2$ zusammen eine Gruppe $-CH_2CH_2-$ oder $-CH_2CH_2CH_2-$bedeuten, sind Bis(2,3-epoxycyclopentyl)ether, 2,3-Epoxycyclopentylglycidylether, 1,2-Bis(2,3-epoxycyclopentyloxy)ethan, 3,4-Epoxy-6-methylcyclohexylmethyl-3',4'-epoxy-6'-methylcyclohexan-carboxylat und 2-(3,4-Epoxy)cyclohexyl-5,5-spiro-(3',4'-epoxy)cyclohexan-dioxan.

Bevorzugt sind Umsetzungsprodukte aus Diglycidylethern von Bisphenol A, Bisphenol F oder Tetrabrom-bisphenol A oder aus Polyglyidylethern von Phenol-Formaldehyd- oder Kresol-Formaldehyd-Novolaken und überschüssigem Bisphenol A, Bisphenol F und/oder Tetrabrom-bisphenol A.

Als Persäuren für die Epoxidierung der Addukte kommen vor allem organische Persäuren, wie z.B. Perameisensäure, Peressigsäure, Perbenzoesäure und Monoperphthalsäure, in Betracht. Die organischen Persäuren können als solche eingesetzt oder in situ gebildet werden, beispielsweise aus alipatischen oder aromatischen Carbonsäuren, Carbonsäureanhydriden, Carbonsäureestern, Säurechloriden oder Keten und Wasserstoffperoxid. Zur in-situ-Bildung der Persäuren verwendet man vorzugsweise aliphatische oder aromatische Mono- oder Dicarbonsäuren oder deren Anhydride, wie Ameisensäure, Essigsäure, Propionsäure, Bernsteinsäureanhydrid, Benzoesäure oder Phthalsäure, und Wasserstoffperoxid, gegebenenfalls unter Zusatz von sauren Katalysatoren, wie Schwefelsäure oder Alkalimetallsalzen. Die Epoxidierung der Addukte wird bevorzugt in Gegenwart von vorgebildeter oder in situ erzeugter Perameisensäure oder Peressigsäure durchgeführt. Gewünschtenfalls können auch anorganische Persäuren, wie Permolybdänsäure, Pervanadinsäure oder Perwolframsäure, eingesetzt werden. Das Epoxidierungsmittel (Persäure) wird zweckmässig in einer Menge von mindestens 1 Mol pro vorhandene Allylgruppe und vorzugsweise im Ueberschuss, z.B. einem 20-200 %igen molaren Ueberschuss, verwendet. So werden bei $R_1$ und/oder $R_3$

= Allyl auch diese Gruppen epoxidiert.

Die Epoxidierung der Addukte wird mit Vorteil in Gegenwart inerter organischer Lösungsmittel und gegebenenfalls unter Zusatz von Puffersubstanzen, wie Natriumacetat oder Natriumhydrogenphosphat, vorgenommen. Als Lösungsmittel eignen sich z.B. gegebenenfalls halogenierte aliphatische oder aromatische Kohlenwasserstoffe, wie Chloroform, Dichlormethan, Benzol, Toluol und Chlorbenzol; Ether, wie Diethylether, Diisopropylether, Dioxan und Tetrahydrofuran, sowie Carbonsäurealkylester, wie Essigsäureethylester und -n-butyl-ester. Halogenierte besonders chlorierte aliphatische Kohlenwasserstoffe sind als Lösungsmittel bevorzugt; besonders bevorzugt ist Chloroform. Die Epoxidierungstemperaturen liegen im allgemeinen zwischen -10 und +100° C, bevorzugt zwischen 10 und 60° C.

Die Verbindungen der Formel I sind bekannt oder können nach an sich bekannten Methoden durch Umsetzung der entsprechenden 2,6-disubstituierten 4-Allyl-, 4-(1-Propenyl) phenole der Formel III

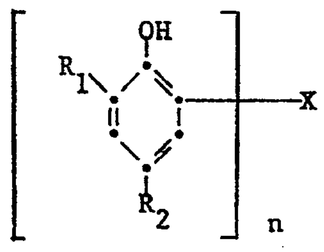

(III)

mit Epihalogenhydrinen, besonders Epichlorhydrin, in Gegenwart von Katalysatoren hergestellt werden. Dabei haben $R_1$, $R_2$, X und n die oben angegebene Bedeutung. Als Phenole der Formel III eignen sich z.B. 4-Allyl-2,6-dimethylphenol, 4-Allyl-2,6-dimethoxyphenol, 4-Allyl-2,6-di-isopropoxyphenol, 4-Allyl-2,6-di-tert.-butylphenol, 2,4-6-Triallylphenol und Bis(2-hydroxy-3-methyl-5-allylphenyl)methan.

Gegenstand der Erfindung sind auch die neuen Addukte aus Verbindungen der Formel I und Verbindungen mit phenolischen OH-Gruppen.

Die Herstellung der Addukte erfolgt auf an sich bekannte Weise in der Schmelze oder einem inerten organischen Lösungsmittel und gegebenenfalls in Gegenwart eines Katalysators. Als inerte Lösungsmittel eignen sich beispielsweise gegebenenfalls chlorierte aromatische Kohlenwasserstoffe, wie Benzol, Toluol und Chlorbenzol; Ketone, wie Aceton, Methylethylketon und Cyclohexanon; höhersiedende Alkohole, wie Butanole, Isopropanol und 2-Ethylhexanol. Die Reaktionstemperaturen liegen im allgemeinen zwischen 100 und 250° C, besonders 120 und 180° C. Bevorzugt ist die Addukt-Bildung in der Schmelze. Als Katalysatoren kommen an sich beliebige, für die Adduktbildung geeignete Verbindungen in Betracht, vor allem Alkalimetallhydroxide, tertiäre Amine, wie Benzyldimethylamin, Tris(dimethylaminomethyl)phenol, Trimethylamin, Triethylamin, Octyldimethylamin, Hexamethylentetramin, sowie gegebenenfalls substituierte Imidazole, wie Imidazol, Benzimidazol, 1-Methylimidazol, 3-Methylimidazol, 2-Ethyl-4-methylimidazol, 2-Phenylimidazol, 2-Phenyl-4-methylimidazol und 1-(2,6-Dichlorbenzoyl)-2-phenylimidazol. Tertiäre Amine, besonders Benzyldimethylamin, und Imidazole, besonders 2-Phenylimidazol, 3-Methylimidazol und 2-Ethyl-4-methylimidazol, sind als Katalysatoren bevorzugt.

Die erfindungsgemäss erhältlichen Polyepoxide sind reine Substanzen, die frei von Chlorid- und Alkalimetallionen sind. Sie eignen sich zur Herstellung von gehärteten Produkten, z.B. für die Umhüllung von integrierten Schaltungen, wofür Produkte von hoher Reinheit benötigt werden. Weiterer Gegenstand der Erfindung sind daher härtbare Gemische, enthaltend

(a) ein erfindungsgemäss erhältliches Polyepoxid und

(b) einen Härter für die Komponente (a).

Dabei können auch Gemische verschiedener erfindungsgemäss erhältlicher Polyepoxide und/oder Härter verwendet werden. Als Härter (b) eignen sich an sich beliebige Epoxidharzhärter, wie z.B. Cyanamid, Dicyandiamid, Polycarbonsäuren, Polycarbonsäureanhydride, Polyamine, Polyaminoamide, Addukte aus Aminen und Polyepoxiden und Polyole.

Geeignete Polycarbonsäuren und ihre Anhydride sind z.B. Phthalsäureanhydrid, Tetrahydro- und Hexahydrophthalsäureanhydrid, Methyltetrahydrophthalsäureanhydrid, Endomethylentetrahydrophthalsäureanhydrid, Hexachlorendomethylentetrahydrophthalsäureanhydrid, Trimellitsäureanhydrid, Pyromellitsäuredianhydrid, Maleinsäureanhydrid, Bernsteinsäureanhydrid, Nonenylbernsteinsäureanhydrid, Dodecenylbernsteinsäureanhydrid, Polysebacinsäurepolyanhydrid und Polyazelainsäurepolyanhydrid sowie die zu den oben genannten Anhydriden gehörenden Säuren.

Als Beispiele von Polyaminen, die sich als Härtungsmittel eignen, seien aliphatische, cycloaliphatische,

aromatische und heterocyclische Polyamine, wie Ethylendiamin, Propan-1,2-diamin, Propan-1,3-diamin, N,N-Diethylethylendiamin, Hexamethylendiamin, Diethylentriamin, Triethylentetramin, Tetraethylenpentamin, N-(2-Hydroxyethyl)-, N-(2-Hydroxypropyl)-und N-(2-Cyanethyl)diethylentriamin, 2,2,4- und 2,4,4-Trimethylhexan-1,6-diamin, m-Xylylendiamin, N,N-Dimethyl- und N,N-Diethylpropan-1,3-diamin, Bis(4-amino-cyclohexyl)methan, 2,2-Bis (4-aminocyclohexyl)propan, 2,2-Bis(4-amino-3-methylcyclohexyl)propan,3-Aminomethyl-3,5,5-trimethylcyclohexylamin (Isophorondiamin), m- und p-Phenylendiamin, Bis(4-aminophenyl)methan, Bis(4-aminophenyl)sulfon, Anilin-Formaldehydharze und N-(2-Aminoethyl)piperazin genannt. Geeignete Polyaminoamide sind z.B. solche, die aus aliphatischen Polyaminen und dimerisierten oder trimerisierten ungesättigten Fettsäuren hergestellt sind.

Als Addukte aus Aminen und Polyepoxiden kommen z.B. Addukte aus aliphatischen oder cycloaliphatischen Diaminen, wie 1,6-Hexamethylendiamin, 2,2,4- und 2,4,4-Trimethylhexan-1,6-diamin oder Isophorondiamin und bekannten Diglycidylethern in Betracht.

Als Polyolhärter (b) kommen vor allem ein- oder mehrkernige aromatische Polyole, einschliesslich Novolake, in Betracht, wie Resorcin, Hydrochinon, 2,6-Dihydroxytoluol, Pyrogallol, 1,1,3-Tris-(hydroxyphenyl)propan, Bis(4,hydroxyphenyl)methan, 2,2-Bis(4-hydroxyphenyl)propan, Bis(4-hydroxyphenyl)sulfon und 4,4'-Dihydroxybiphenyl sowie Novolake aus Formaldehyd oder Acetaldehyd und Phenol, Chlorphenol oder Alkylphenolen mit bis zu 9 C-Atomen im Alkyl, besonders Kresol- und Phenolnovolake.

Bevorzugte Härter sind Polycarbonsäureanhydride, wie Tetrahydro-, Hexahydro- und Methyltetrahydrophthalsäureanhydrid, sowie aromatische Polyamine, besonders Bis(4-aminophenyl)methan, Bis(4-aminophenyl)sulfon und m- oder p-Phenylendiamin.

Die Härter (b) werden in den in der Epoxidharztechnik üblichen Mengen eingesetzt, zweckmässig in solchen Mengen, dass auf ein Epoxidäquivalent etwa 0,7 bis 1,5 Aequivalente funktioneller Gruppen des Härters (b) kommen.

Die erfindungsgemässen Gemische können auch weitere übliche Zusätze enthalten, vor allem (c) Beschleuniger bzw. Härtungskatalysatoren, und/oder (d) weitere Epoxidharze.

Als Beschleuniger (c) können ebenfalls an sich bekannte Verbindungen verwendet werden. Als Beispiele seien genannt: Komplexe von Aminen, besonders tertiären Aminen, wie Monoethylamin, Trimethylamin und Octyldimethylamin, mit Bortrifluorid oder Bortrichlorid, tertiäre Amine, wie Benzyldimethylamin, Tris-(dimethylaminomethyl)phenol, Hexamethylentetramin oder 1,6-Bis(dimethylamino)hexan; Harnstoffderivate, wie N-4-Chlorphenyl-N',N'-dimethylharnstoff (Monuron), N-3-Chlor-4-methylphenyl-N',N'-dimethylharnstoff (Chlortoluron), N-(2-Hydroxyphenyl)-N',N'-dimethylharnstoff und N-(2-Hydroxy-4-nitrophenyl)-N',N'-dimethylharnstoff und gegebenenfalls substituierte Imidazole, wie Imidazol, Benzimidazol, 1-Methylimidazol, 2-Ethyl-4-methylimidazol, 2-Vinylimidazol, 2-Phenylimidazol, 2-Phenyl-4-methylimidazol, 1-(2,6-Dichlorbenzoyl)-2-phenylimidazol und 1-(2,4,6-Trimethylbenzoyl)-2-phenylimidazol. Tertiäre Amine, besonders Benzyldimethylamin, und Imidazole, besonders 2-Phenylimidazol, 3-Methylimidazol und 2-Ethyl-4-methylimidazol, sind als Beschleuniger (c) bevorzugt.

Als weitere Epoxidharze (d) kommen vor allem solche mit durchschnittlich mehr als einer an ein Heteroatom, z.B. an ein S- und vorzugsweise an ein O- oder N-Atom, gebundenen Gruppe der Formel II, wie sie im Vorangehenden beschrieben sind, in Betracht.

Besonders bevorzugt setzt man als Komponente (d) gegebenenfalls vorverlängerte Diglycidylether von zweiwertigen Phenolen oder Cyclohexanolen, vor allem 2,2-Bis(4-hydroxyphenyl)propan, 2,2-Bis(3,5-dibrom-4-hydroxyphenyl)-propan, Bis(4-hydroxyphenyl)methan, Bis(4-hydroxycyclohexyl)methan oder 2,2-Bis(4-hydroxycyclohexyl)propan, Polyglycidylether von Novolaken, oder tetraglycidyliertes 4,4'-Diaminodiphenylmethan ein. Ganz besonders bevorzugt sind gegebenenfalls vorverlängerte Diglycidylether von Bisphenol A, Tetrabrom-bisphenol A oder Bisphenol F, Polyglycidylether von Phenol-Formaldehyd- oder Kresol-Formaldehyd-Novolaken, oder Gemische davon.

Die Komponenten (b) und (c) werden in den üblichen wirksamen, d.h. für die Härtung der erfindungsgemässen Gemische ausreichenden Mengen eingesetzt. Das Verhältnis der Komponenten (a), (b), (c) und gegebenenfalls (d) hängt von der Art der verwendeten Verbindungen, der erforderlichen Härtungsgeschwindigkeit und den im Endprodukt gewünschten Eigenschaften ab und kann vom Fachmann auf dem Gebiet der Epoxidharz-Härtung leicht ermittelt werden. Wenn das Härtungsmittel (b) ein Amin ist, werden normalerweise 0,75 bis 1,25 Aequivalente Aminwasserstoff pro 1 Epoxidäquivalent eingesetzt. Bei Polycarbonsäure- oder Polycarbonsäureanhydrid-Härtern verwendet man gewöhnlich 0,4 bis 1,1 Aequivalente Carboxyl- bzw. Anhydridgruppen pro 1 Epoxidäquivalent. Bei der Verwendung von Polyphenolen als Härtungsmittel setzt man zweckmässig 0,75 bis 1,25 phenolische Hydroxylgruppen pro 1 Epoxidäquivalent ein. Beschleuniger (c) werden im allgemeinen in Mengen von 0,1 bis 5 Gewichtsprozent, bezogen die Epoxidharze (a) und gegebenenfalls (d), verwendet.

Gewünschtenfalls kann man den härtbaren Gemischen zur Herabsetzung der Viskosität reaktive

Verdünner, wie z.B. Styroloxid, Butylglycidylether, 2,2,4-Trimethylpentylglycidylether, Phenylglycidylether, Kresylglycidylether oder Glycidylester von synthetischen, hochverzweigten, in der Hauptsache tertiären aliphatischen Monocarbonsäuren, zusetzen. Als weitere übliche Zusätze können die erfindungsgemässen Gemische ferner Weichmacher, Streck-, Füll- und Verstärkungsmittel, wie beispielsweise Steinkohlenteer, Bitumen, Textilfasern, Glasfasern, Asbestfasern, Borfasern, Kohlenstoff-Fasern, mineralische Silikate, Glimmer, Quarzmehl, Aluminiumoxidhydrat, Betonite, Kaolin, Kieselsäureaerogel oder Metallpulver, z.B. Aluminiumpulver oder Eisenpulver, ferner Pigmente und Farbstoffe, wie Russ, Oxidfarben und Titandioxid, Flammschutzmittel, Thixotropiemittel, Verlaufmittel ("flow control agents"), wie Silicone, Wachse und Stearate, die zum Teil auch als Formtrennmittel Anwendung finden, Haftvermittler, Antioxidantien und Lichtschutzmittel enthalten.

Die erfindungsgemässen Gemische finden z.B. Anwendung als Klebstoffe oder zur Herstellung von gehärteten Produkten, wie Verbundwerkstoffen und Laminaten, insbesondere jedoch zum Umhüllen elektronischer Bauteile. Sie können in jeweils dem speziellen Anwendungsgebiet angepasster Formulierung, in ungefülltem oder gefülltem Zustand, z.B. als Anstrichmittel, Beschichtungsmassen, Lacke, Pressmassen, Tauchharze, Giessharze, Imprägnierharze, Laminierharze, Matrixharze und Klebemittel verwendet werden.

Die Härtung der erfindungsgemässen Gemische kann auf an sich bekannte Weise ein- oder zweistufig vorgenommen werden. Die Härtung der erfindungsgemässen Gemische erfolgt im allgemeinen durch Erhitzen auf Temperaturen zwischen 80 und 200°C, besonders 100 und 180°C.

Die mit den erfindungsgemäss erhältlichen Polyepoxiden hergestellten gehärteten Produkte zeichnen sich durch gute mechanische, thermische und chemische Eigenschaften aus.

Die Erfindung wird durch die folgenden Beispiele näher erläutert.

A. Herstellung des Ausgangsprodukts (4-Allyl-2,6-dimethylphenylglycidylether)

In einem Reaktionsgefäss aus Glas, versehen mit Dosiervorlage, Rührer, Thermomether und einem Azeotropdestillationsaufsatz mit Rückflusskühler und Vakuumanschluss werden 350 g (1 Mol) 4-Allyl-2,6-dimethylphenol, 1796 g (19,42 Mol) Epichlorhydrin und 16,20 g Tetramethylammoniumchlorid zusammengemischt, auf 60°C erwärmt und 2 Stunden bei 60°C gerührt. Durch Anlegen eines Vakuums von 6700 Pa wird das Epichlorhydrin bei der Reaktionstemperatur (ca. 60°C) zum Rückfluss gebracht. Im Verlaufe von 3,5 Std. werden tropfenweise 190 g einer 50%igen wässrigen NaOH-Lösung zugegeben, wobei die Innentemperatur bei 60°C gehalten wird. Während der Reaktion wird das gebildete Wasser mit dem siedenden Epichlorhydrin abdestilliert. Nach der Zugabe der Natronlauge wird das Reaktionsgemisch noch etwa 2 Std. weitergerührt. Nach der Beendigung der Reaktion wird das gebildete Kochsalz abfiltriert, und das Filtrat wird mit 1 Liter 10%iger $Na_2SO_4$-Lösung neutralisiert. Die organische Phase wird abgetrennt und zweimal mit je 600 ml destilliertem Wasser gewaschen, von der wässrigen Phase getrennt und mit Natriumsulfat getrocknet. Anschliessend wird das überschüssige Epichlorhydrin mit Hilfe eines Rotationsverdampfers bei 60°C/2660 Pa abdestilliert. Die Destillation des Rückstands bei 110°C/1,3 Pa ergibt 358 g (75,5 % d. Th.) 4-Allyl-2,6-dimethylphenylglycidylether; Epoxidgehalt 4,54 Aequivalente/kg.

| Elementaranalyse: | | |
|---|---|---|
| berechnet | C 76,68 % | H 8,73 % |
| gefunden | C 76,52 % | H 8,43 %. |

$^1$H-NMR-Spektrum: 2,2 ppm (s) 6H ($CH_3$), ca. 2,75 ppm (m) 2H

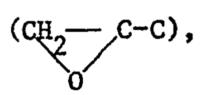

ca. 3,3 ppm (m) 3H

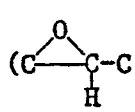 und 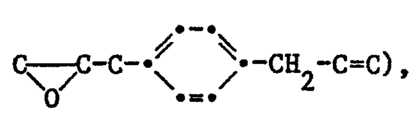

ca. 3,85 ppm (m) 2H

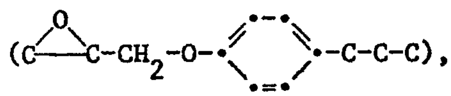

5,1 ppm (m) 2H ($CH_2=C-C$), 6,0 ppm (m) 1H

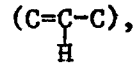

6,85 ppm (m) 2H

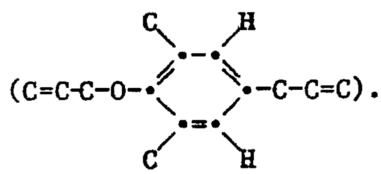

Herstellung der Addukte

Beispiel 1: 30,84 g (0,1 phenolische OH-Aequivalente) 2,4,6-Triallylphenol (Verbindung B) und 43,86 g (0,1 Epoxidäquivalent) 4-Allyl-2,6-dimethylphenylglycidylether (Verbindung A) werden gemischt und bei 80 °C gelöst. 0,43 g 2-Phenylimidazol werden in dem erhaltenen rotbraunen Gemisch gelöst, und das Gemisch wird auf 180 °C erhitzt. Anschliessend wird das Gemisch 2 Stunden bei 180 °C gehalten. Dabei sinkt der Epoxidgehalt des gebildeten Addukts auf etwa 0,15 Aequivalente/kg. Das Addukt hat eine Schmelzviskosität von etwa 116 mPa s bei 100 °C.

Beispiele 2-5: Auf die in Beispiel 1 beschriebene Weise werden weitere Addukte aus der Verbindung A und den folgenden Polyolen bzw. Novolaken hergestellt:
Beispiel 2: o-Kresolnovolak mit 8,3 OH-Aequivalenten/kg
Beispiel 3: 2,2-Bis(4-hydroxy-3,5-diallylphenyl)propan
Beispiel 4: 2,2-Bis(4-hydroxy-3-allylphenyl)propan
Beispiel 5: Bis(2-hydroxy-3-methyl-5-allylphenyl)methan.

Die Reaktionsbedingungen und die Schmelzviskositäten der erhaltenen Produkte sind in der folgenden Tabelle I angegeben.

Tabelle I

| Bei-spiel Nr. | Verbindung A (g) | Verbindung B (g) | 2-Phenylimidazol (g) | Reaktionszeit/-temperatur Std./°C | Schmelzviskosität mPa s/100°C |
|---|---|---|---|---|---|
| 2 | 87,7 | 61,6 g o-Kresolnovolak | 0,015 | 4/175 | 206 |
| 3 | 21,83 | 21,05 g 2,2-Bis(4-hydroxy-3,5-diallylphenyl)propan | 0,22 | 3/166 | 210 |
| 4 | 21,83 | 21,42 g 2,2-Bis(4-hydroxy-3-allylphenyl)propan | 0,22 | 4/170 | 10 |
| 5 | 43,8 | 24,1 g Bis(2-hydroxy-3-methyl-5-allylphenyl)methan | 0,0024 | 6/180 | |

Epoxidierung der Addukte

Beispiel 6: 37,5 g (0,087 Mol) des gemäss Beispiel 1 hergestellten Addukts aus 4-Allyl-2,6-dimethylphenylglycidylether und 2,4,6-Triallylphenol in 50 ml Chloroform und 0,1 g Natriumacetat werden in einem

350 ml Sulfierkolben, der mit Rührer, Kühler, Tropftrichter und Thermometer ausgerüstet ist, vorgelegt. Innerhalb von etwa 2 Stunden werden bei 30-35 °C 66,12 g (0,348 Mol) 40%ige Peressigsäure zugetropft. Das Reaktionsgemisch wird während 6 Std. weitergerührt, anschliessend in einen Scheidetrichter übergeführt, zuerst mit 3%iger NaOH und dann mit Wasser gewaschen, mit $Na_2SO_3$ peroxidfrei gestellt, über Natriumsulfat getrocknet und eingeengt. Nach dem Einengen erhält man 27,63 g (63,96 % d.Th.) eines hochviskosen Harzes mit einem Epoxidgehalt von 4,539 Aequivalenten/kg (etwa 56,3 % d.Th.). Aus dem Gelchromatogramm werden die folgenden Molekulargewichte ermittelt:

$M_n$ (Zahlenmittel) = 570

$M_w$ (Gewichtsmittel) = 814.

Beispiel 7: In einem 200 ml Sulfierkolben, der mit Rührer, Kühler, Tropftrichter und Thermometer ausgestattet ist, werden 62,0 g (182 mMol) des gemäss Beispiel 2 hergestellten Adduktes aus 4-Allyl-2,6-dimethylphenylglycidylether und o-Kresolnovolak in 50 ml Chloroform vorgelegt. Innerhalb von 90 Minuten werden bei 30-35 °C 41,80 g (220 mMol) 40%ige Peressigsäure zugetropft. Nach Beendigung des Zutropfens wird das Reaktionsgemisch noch während 16 Std. gerührt. Zum Aufarbeiten wird das erhaltene Produkt mit 3%iger NaOH und dann mit wässriger Kochsalz-Lösung gewaschen, mit $Na_2SO_3$ peroxidfrei gestellt, über Natriumsulfat getrocknet und eingeengt. Nach dem Einengen erhält man 35,16 g eines festen Epoxidharzes mit einem Epoxidgehalt von 2,372 Aequivalenten/kg.

Beispiel 8: In einem 200 ml Sulfierkolben, der mit Rührer, Kühler, Thermometer und Tropftrichter ausgestattet ist, werden 30,0 g (0,036 Mol) des gemäss Beispiel 3 hergestellten Addukts aus 4-Allyl-2,6-dimethylphenylglycidylether und 2,2-Bis(4-hydroxy-3,5-diallylphenyl)propan in 50 ml Chloroform und 1,0 g Natriumacetat vorgelegt. Innerhalb von 90 Minuten werden bei 30-35 °C 41,80 g (0,22 Mol) 40%ige Peressigsäure zugetropft. Das Reaktionsgemisch wird noch während weiterer 6 Std. gerührt und dann in einen Scheidetrichter übergeführt. Das erhaltene Reaktionsprodukt wird mit 3%iger NaOH-Lösung und NaCl-Lösung gewaschen, mit Natriumsulfit peroxidfrei gestellt, über Natriumsulfat getrocknet und eingeengt. Nach dem Entfernen des Lösungsmittels erhält man 27,65 g (92,2 % d.Th.) eines hochviskosen Epoxidharzes mit einem Epoxidgehalt von 5,097 Aequivalenten/kg (70 % d.Th.).

Beispiel 9: In einem 350 ml Sulfierkolben, der mit Rührer, Thermometer, Kühler und Tropftrichter ausgestattet ist, werden 50 g des gemäss Beispiel 4 hergestellten Addukts aus 4-Allyl-2,6-dimethylphenylglycidylether und 2,2-Bis(4-hydroxy-3-allylphenyl)propan in 50 ml Chloroform vorgelegt. Innerhalb von etwa 120 Minuten werden bei 30-35 °C 54,96 g (0,29 Mol) 40%ige Peressigsäure zugetropft. Nach weiteren 4 Std. Rühren wird das Reaktionsgemisch in einen Scheidetrichter übergeführt, mit 3%iger NaOH und Wasser gewaschen, mit Natriumsulfit peroxidfrei gestellt, über Natriumsulfat getrocknet und eingeengt. Nach dem Einengen erhält man 43,12 g eines bräunlichen festen Epoxidharzes mit einem Epoxidgehalt von 3,46 Aequivalenten/kg.

Beispiel 10: In einem 200 ml Sulfierkolben, der mit Tropftrichter, Rührer, Kühler und Thermometer ausgestattet ist, werden 40,0 g (0,054 Mol) des gemäss Beispiel 5 hergestellten Adduktes aus 4-Allyl-2,6-dimethylphenylglycidylether und Bis(2-hydroxy-3-methyl-5-allylphenyl)methan in 50 ml Chloroform und 1,0 g Natriumacetat vorgelegt. Innerhalb von etwa 2 Std. werden bei 30-35 °C 66,12 g (0,348 Mol) 40%ige Peressigsäure zugetropft. Nach weiteren 5-6 Std. Rühren wird das Reaktionsprodukt nach dem in Beispiel 6 angegebenen Verfahren aufgearbeitet. Man erhält 43,2 g (98,64 % d.Th.) eines hochviskosen Epoxidharzes mit einem Epoxidgehalt von 3,65 Aequivalenten/kg (73,94 % d.Th.).

Anwendungsbeispiele I-V

Die gemäss den Beispielen 7, 8 und 10 erhaltenen Epoxidharze werden mit einem Härter und einem Härtungsbeschleuniger (2-Ethyl-4-methylimidazol) vermischt und zu gehärteten Formkörpern verarbeitet. In der folgenden Tabelle II sind Art und Menge der verwendeten Komponenten, die Härtungsbedingungen und die Eigenschaften der gehärteten Formkörper zusammengefasst.

Tabelle II

| Anwendungsbeispiel Nr. | Epoxidharz gemäss Beispiel Nr. | g | Härter [1] (g) | 2-Ethyl-4-methylimidazol (g) | Härtungsbedingungen Zeit/Temp. | Eigenschaften des gehärteten Formkörpers Tg [2] |
|---|---|---|---|---|---|---|
| II | 7 | 41,82 | 18,07 | 0,04 | 4 h/180°C | 134°C |
| III | 8 | 26,46 | 15,66 | 0,13 | 2 h/120°C<br>2 h/150°C<br>2 h/180°C | 142°C |
| V | 10 | 8,44 | 2,41 | 0,008 | 4 h/180°C | 116°C |

1) Härter = o-Kresolnovolak mit 8,3 Aequivalenten phenolische OH-Gruppen/kg

2) bestimmt mittels DSC (Differential Scanning Calorimetry) auf einem Gerät Mettler TA 3000

## Patentansprüche

1. Polypoxide, die dadurch erhältlich sind, dass man Verbindungen der Formel I

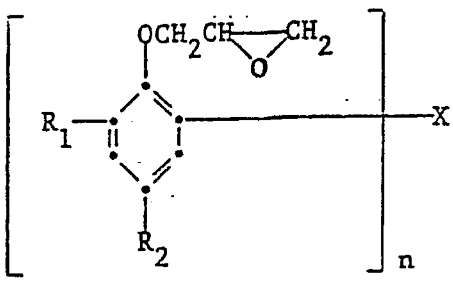

(I),

worin n die Zahl 1 oder 2 ist, X bei n = 1 eine Gruppe $R_3$ und bei n = 2 eine Gruppe der Formel

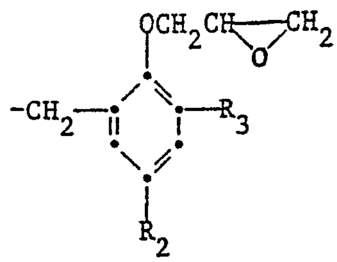

darstellt,
$R_1$ und $R_3$ unabhängig voneinander $C_{1-4}$-Alkyl, $C_{1-4}$-Alkoxy, Allyl, ein Halogenatom oder $C_{6-10}$-Aryl und $R_2$ Allyl oder 1-Propenyl oder bedeuten, mit Verbindungen mit phenolischen OH-Gruppen umsetzt und die erhaltenen Addukte anschliessend in Gegenwart einer Persäure epoxidiert.

2. Polyepoxide nach Anspruch 1, worin $R_1$ und $R_3$ unabhängig voneinander $C_{1-4}$-Alkyl, $C_{1-2}$-Alkoxy, ein Halogenatom, besonders ein Chloratom, oder Phenyl und $R_2$ Allyl bedeuten.

3. Polyepoxide nach Anspruch 1, worin $R_1$ und $R_3$ dieselbe Bedeutung haben und $R_2$ Allyl darstellt.

4. Polyepoxide nach Anspruch 1, worin n = 1 ist.

5. Polyepoxide nach Anspruch 3, worin n = 1 ist, $R_1$ und $R_3$ je Methyl, tert.-Butyl, Methoxy, Chlor oder Phenyl und $R_2$ Allyl bedeuten.

6. Polyepoxide nach Anspruch 5, worin n = 1 ist, $R_1$ und $R_3$ je Methyl und $R_2$ Allyl bedeuten.

7. Polyepoxide nach Anspruch 1, dadurch gekennzeichnet, dass man als Verbindungen mit phenolischen OH-Gruppen ein- oder mehrkernige Mono- oder Polyphenole verwendet.

8. Polyepoxide nach Anspruch 7, dadurch gekennzeichnet, dass man als Mono- oder Polyphenol Bisphenol A, Bisphenol F, Tetrabrombisphenol A, Triallylphenol, 2,2-Bis(4-hydroxy-3-allylphenyl)propan, 2,2-Bis(4-hydroxy-3,5-diallylphenyl)propan, einen Phenolformaldehyd- oder Kresolformaldehyd-Novolak verwendet.

9. Polyepoxide nach Anspruch 1, dadurch gekennzeichnet, dass man als Verbindungen mit phenolischen OH-Gruppen Umsetzungsprodukte aus einem Polyepoxid und einem Ueberschuss an Polyphenolen verwendet.

10. Polyepoxide nach Anspruch 9, dadurch gekennzeichnet, dass man Umsetzungsprodukte aus Diglycidylethern von Bisphenol A, Bisphenol F oder Tetrabrombisphenol A oder aus Polyglyidylethern von Phenolformaldehyd- oder Kresolformaldehyd-Novolaken und überschüssigem Bisphenol A, Bisphenol F und/oder Tetrabrombisphenol A verwendet.

11. Polyepoxide nach Anspruch 1, dadurch gekennzeichnet, dass man die Epoxidierung in Gegenwart von

Perameisensäure oder Peressigsäure vornimmt.

**12.** Addukte aus Verbindungen der Formel I

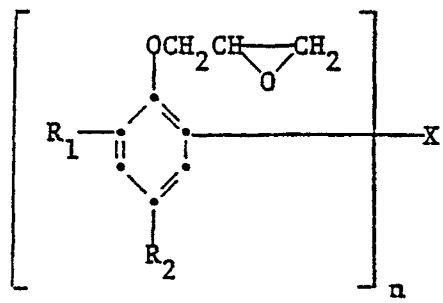

(I),

worin n die Zahl 1 oder 2 ist, X bei n = 1 eine Gruppe $R_3$ und bei n = 2 eine Gruppe der Formel

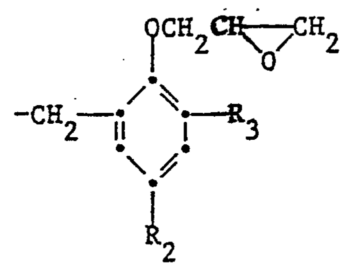

darstellt,
$R_1$ und $R_3$ unabhängig voneinander $C_{1-4}$-Alkyl, $C_{1-4}$-Alkoxy, Allyl, ein Halogenatom oder $C_{6-10}$-Aryl und
$R_2$ Allyl oder 1-Propenyl bedeuten, und Verbindungen mit phenolischen OH-Gruppen.

**13.** Härtbare Gemische enthaltend
(a) ein erfindungsgemäss erhältliches Polyepoxid nach Anspruch 1 und
(b) einen Härter für die Komponente (a).

**14.** Härtbare Gemische nach Anspruch 13, die zusätzlich
(c) einen Härtungsbeschleuniger enthalten.

**15.** Härtbare Gemische nach Anspruch 13 oder 14, die zusätzlich
(d) ein weiteres Epoxidharz enthalten.

**16.** Verwendung von härtbaren Gemischen nach Anspruch 13 als Klebstoffe oder zur Herstellung von gehärteten Produkten, besonders zum Umhüllen elektronischer Bauteile.

## Claims

**1.** A polyepoxide which is obtainable by reacting a compound of the formula I

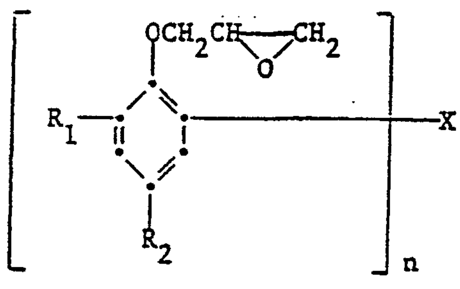

(I)

in which n is the number 1 or 2, and, if n is 1, X is a group $R_3$, or, if n is 2, X is a group of the formula

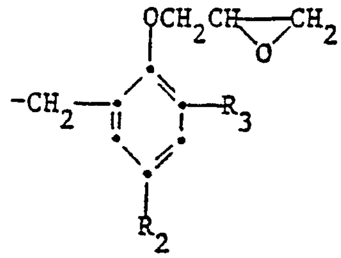

each of $R_1$ and $R_3$ independently of the other is $C_1$-$C_4$alkyl, $C_1$-$C_4$alkoxy, allyl, a halogen atom or $C_6$-$C_{10}$aryl and $R_2$ is allyl or 1-propenyl, with a compound having phenolic OH groups, and subsequently epoxidising the resultant adduct in the presence of a peracid.

**2.** A polyepoxide according to claim 1, in which each of $R_1$ and $R_3$ independently of the other is $C_1$-$C_4$alkyl, $C_1$-$C_2$alkoxy, a halogen atom, in particular a chlorine atom, or phenyl and $R_2$ is allyl.

**3.** A polyepoxide according to claim 1, in which $R_1$ and $R_3$ have the same meaning and $R_2$ is allyl.

**4.** A polyepoxide according to claim 1, in which n is 1.

**5.** A polyepoxide according to claim 3, in which n is 1, each of $R_1$ and $R_3$ is methyl, tert-butyl, methoxy, chlorine or phenyl and $R_2$ is allyl.

**6.** A polyepoxide according to claim 5, in which n is 1, each of $R_1$ and $R_3$ is methyl and $R_2$ is allyl.

**7.** A polyepoxide according to claim 1, wherein a mono- or polynuclear mono- or polyphenol is used as the compound having phenolic OH groups.

**8.** A polyepoxide according to claim 7, wherein bisphenol A, bisphenol F, tetrabromobisphenol A, triallylphenol, 2,2-bis(4-hydroxy-3-allylphenyl)propane, 2,2-bis(4-hydroxy-3,5-diallylphenyl)propane, a phenol/formaldehyde or cresol/formaldehyde novolak is used as the mono-or polyphenol.

**9.** A polyepoxide according to claim 1, wherein a reaction product of a polyepoxide and an excess of polyphenol is used as the compound having phenolic OH groups.

**10.** A polyepoxide according to claim 9, wherein a reaction product of a diglycidyl ether of bisphenol A, bisphenol F or tetrabromobisphenol A or of a polyglycidyl ether of a phenol/formaldehyde or cresol/formaldehyde novolak and excess bisphenol A, bisphenol F and/or tetrabromobisphenol A is used.

**11.** A polyepoxide according to claim 1, wherein the epoxidation is carried out in the presence of performic acid or peracetic acid.

**12.** An adduct of a compound of the formula I

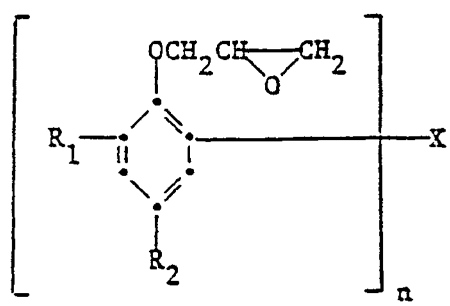

(I)

in which n is the number 1 or 2, and, if n is 1, X is a group $R_3$, or, if n is 2, X is a group of the formula

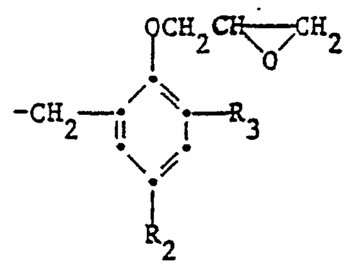

each of $R_1$ and $R_3$ independently of the other is $C_1$-$C_4$alkyl, $C_1$-$C_4$alkoxy, allyl, a halogen atom or $C_6$-$C_{10}$aryl and $R_2$ is allyl or 1-propenyl, with a compound having phenolic OH groups.

**13.** A curable mixture containing
(a) a polyepoxide according to claim 1 obtainable according to the invention and
(b) a hardener for the component (a).

**14.** A curable mixture according to claim 13, which additionally contains
(c) a curing accelerator.

**15.** A curable mixture according to either of claims 13 or 14, which additionally contains (d) a further epoxy resin.

**16.** Use of a curable mixture according to claim 13 as an adhesive or for the preparation of cured products, in particular for coating electronic components.

**Revendications**

**1.** Polyépoxydes que l'on obtient en faisant réagir des composés de formule I ci-dessous :

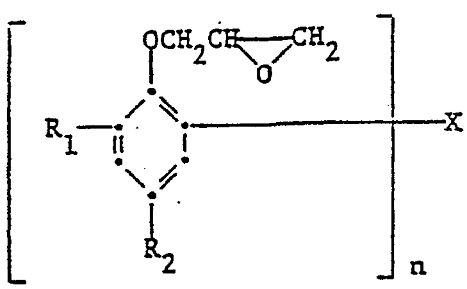

(I),

(dans laquelle n est le nombre 1 ou 2, X, si n = 1, est un groupe $R_3$ tel que ci-dessous, et si n = 2 un groupe de formule :

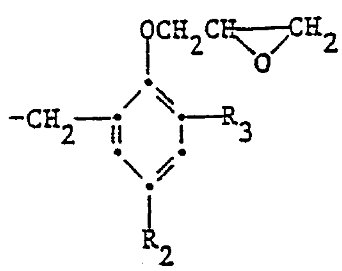

$R_1$ et $R_3$ sont chacun, indépendamment l'un de l'autre, un alkyle ou un alcoxy en $C_{1-4}$, un allyle ou atome d'halogène ou un aryle en $C_{6-10}$, et $R_2$ est le groupe allyle ou 1-propényle) avec des composés

à groupes OH phénoliques, puis en époxydant les produits d'addition ainsi formés avec un peracide.

2. Polyépoxydes selon la revendication 1 dans lesquels $R_1$ et $R_3$ sont chacun un alkyle en $C_{1-4}$, un alcoxy en $C_{1-2}$, ou bien un atome d'halogène, notamment de chlore, ou le groupe phényle, et $R_2$ est le groupe allyle.

3. Polyépoxydes selon la revendication 1 dans lesquels $R_1$ et $R_3$ sont identiques et $R_2$ est le groupe allyle.

4. Polyépoxydes selon la revendication 1 dans lesquels n = 1.

5. Polyépoxydes selon la revendication 3 dans lesquels n = 1, $R_1$ et $R_3$ sont chacun le groupe méthyle, tert-butyle, méthoxy ou phényle ou un atome de chlore et $R_2$ est le groupe allyle.

6. Polyépoxydes selon la revendication 5 dans lesquels n = 1, $R_1$ et $R_3$ sont chacun le groupe méthyle et $R_2$ le groupe allyle.

7. Polyépoxydes selon la revendication 1 caractérisés en ce qu'ils sont formés avec des monophénols ou polyphénols monocycliques ou polycycliques comme composés à groupes hydroxyliques phénoliques.

8. Polyépoxydes selon la revendication 7 caractérisés en ce qu'ils sont formés avec, comme monophénols ou polyphénols, le bisphénol A, le bisphénol F, le tétrabromo-bisphénol A, le triallylphénol, le 2,2-bis-(4-hydroxy-3-allylphényl)-propane, le 2,2-bis-(4-hydroxy-3,5-diallylphényl)-propane ou avec une novolaque phénol-formaldéhyde ou crésol-formaldéhyde.

9. Polyépoxydes selon la revendication 1, caractérisés en ce qu'ils sont formés avec, comme composés à hydroxyles phénoliques, des produits de réaction d'un polyépoxyde avec un excès de polyphénols.

10. Polyépoxydes selon la revendication 9, caractérisés en ce qu'ils sont formés avec des produits de réaction d'éthers diglycidyliques du bisphénol A, du bisphénol F ou du tétrabromo-bisphénol A, ou bien d'éthers polyglycidyliques de novolaques phénol-formaldéhyde ou crésol-formaldéhyde, avec un excès de bisphénol A, de bisphénol F et/ou de tétrabromobisphénol A.

11. Polyépoxydes selon la revendication 1, caractérisés en ce qu'ils sont obtenus par époxydation avec de l'acide performique ou peracétique.

12. Produits d'addition de composés de formule I :

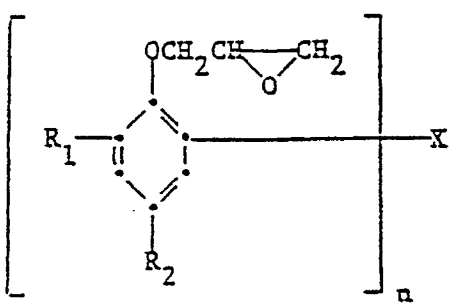

(I),

(dans laquelle n est le nombre 1 ou 2, X, si n = 1, est un groupe $R_3$ tel que ci-dessous, et si n = 2 un groupe de formule :

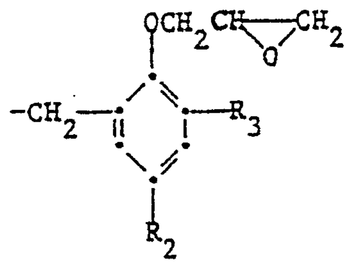

$R_1$ et $R_3$ représentent chacun, indépendamment l'un de l'autre, un alkyle ou un alcoxy en $C_{1-4}$ ou bien le groupe allyle, un atome d'halogène ou un aryle en $C_{6-10}$, et $R_2$ est le groupe allyle ou 1-propényle) et de composés à groupes hydroxyliques phénoliques.

**13.** Mélanges durcissables qui comprennent :
(a) un polyépoxyde selon la revendication 1 avec
(b) un durcisseur de ce composant (a).

**14.** Mélanges durcissables selon la revendication 13 qui contiennent en outre (c) un accélérateur de durcissement.

**15.** Mélanges durcissables selon la revendication 13 ou 14 qui contiennent en outre (d) une autre résine époxyde.

**16.** L'emploi des mélanges durcissables de la revendications 13 comme adhésifs ou pour fabriquer des produits durcis, et en particulier pour l'enrobage de composants et éléments électroniques.